# EUROPEAN PATENT APPLICATION

(11) **EP 3 772 741 A1**
(43) Date of publication of application: **10.02.2021**
(21) Application number: 19190310.3
(22) Date of filing: 06.08.2019
(51) Int. Cl.: H01G 4/005, H01G 4/228, H01G 2/10, H01G 4/224, H01G 9/04, H01G 9/08, H01G 9/008, H01G 9/14, H01G 11/74, H01G 11/78, A61N 1/18, A61N 1/378

(54) **CAPACITOR WITH MULTIPLE ELECTRODES**

(71) Applicant: BIOTRONIK SE & Co. KG, 12359 Berlin (DE)
(72) Inventor: Dörr, Thomas, 12437 Berlin (DE); Weiss, Ingo, 12435 Berlin (DE)
(74) Representative: Galander, Marcus

(57) **Abstract**

The present invention relates to a capacitor comprising at least a first electrode having a first polarity, at least two second electrodes having a second polarity opposite to the first polarity, and a housing accommodating the at least first electrode and the at least two second electrodes, wherein each of the at least first electrode and the at least two second electrodes are electrically contactable separately from each other from the outside of the housing.

## Description

The present invention relates to a capacitor with multiple electrodes.

Commonly, capacitors or condensers are passive two-terminal electronic components that can store electrical energy in an electric field and are broadly used in electronic devices.

In particular in implantable cardioverter defibrillators (ICD), capacitors are mandatory to provide a current with sufficient voltage and strength for the therapeutic shock, since the typically built-in batteries are not able to provide the required currents. Frequently, tantalum electrolytic capacitors are used in ICDs due to their presently superior capacitance and dielectric strength. However, up to three of those capacitors are needed in an ICD to provide sufficient power for the therapeutic treatment, encumbering the manufacture of the ICD and increasing the work labour and costs. Additionally, such tantalum electrolytic capacitors or electrolytic capacitors in general are not variable or trimmable.

Based on the above, there is still a need for capacitors with increased energy density, preferably with a tuneable capacitance to advantageously reduce the complexity of an ICD layout, e.g. in terms of number of necessary components, etc.

Accordingly, it is an objective of the present invention to provide a capacitor with increased energy density and tuneable capacitance, and an implantable medical device utilizing such capacitor.

This objective is attained by a capacitor having the features of claim 1 and an implantable medical device having the features of claim 14. Appropriate embodiments thereof are stated in the dependent claims and the following description.

According to claim 1, a capacitor is provided, wherein the capacitor comprises
- at least a first electrode having a first polarity,
- at least two second electrodes having a second polarity opposite to the first polarity, and
- a housing accommodating the at least first electrode and the at least two second electrodes,
wherein
each of the at least first electrode and the at least two second electrodes are electrically contactable separately from each other from the outside of the housing.

Advantageously, by contacting each of the electrodes separately from each other, a plurality of capacitances is achievable within the capacitor of the invention, e.g. by individually contacting each of the electrodes and/or by parallel connecting two or more electrodes having the second polarity.

Preferably, each of the at least first electrode and at least two second electrodes is formed such the at least first electrode corresponds in terms of the overlapping surface with at least one of the at least two second electrodes.

In some embodiments of the capacitor of the invention, the at least first electrode or the at least two electrodes are electrically contactable form the outside of the housing via at least one feedthrough extending through the housing.

The term "feedthrough" in the context of the present specification is used in its meaning known in the art; it particularly refers to an electrically connecting element, which is surrounded by an insulating body, which insulates the electrically connecting element from other electrically connecting elements or the housing. The feedthrough may further comprise a flange surrounding the insulating body. The electrically connecting element may be a wire or a pin electrically connected with, particularly joined with, the respective electrode.

The insulating body or the flange may be joined to the housing by appropriate techniques such as welding, brazing, soldering, adhesive bonding, crimping or flanging. Likewise, the insulating body and the flange may be joined by any one of the aforementioned techniques.

The insulating body may have more the one aperture for guiding more than one electrically connecting elements through the housing. Accordingly, two or more second electrodes having the second polarity may be guided through the housing by one feedthrough, wherein each of the second electrodes are separated or electrically insulated from each other, e.g., by a common insulating body. Alternatively, each of the second electrodes may have an own feedthrough.

In some embodiments of the capacitor of the invention, the housing is made of an insulating material, wherein particularly the insulating body of the feedthrough may be advantageously formed by the housing.

In some embodiments of the capacitor of the invention, the feedthrough comprises an insulting body made of a glass, a ceramic or a polymer, particularly an LCP or an epoxy resin, or a composite thereof.

In some embodiments of the capacitor of the invention, the feedthrough is hermetically sealed. This is particularly important in case of the capacitor of the invention is an electrolytic capacitor, wherein the electrolyte is sealed within the housing, which is preferably hermetically sealed too.

Accordingly in some embodiments of the capacitor of the invention, the housing is hermetically sealed, i.e. gas-tight and/or fluid-tight. Particularly, the housing is resistant against a pressure difference of at least 2 bar, preferable a pressure difference in the range of 2 bar to 10 bar.

In some embodiments of the capacitor of the invention, the housing is made of an electrically conductive material and in electrically conductive communication with the at least first electrode, wherein the at least two second electrodes are electrically contactable from the outside of the housing by at least one feedthrough, and each of the at least two second electrode are electrically insulated from each other electrode and from the housing.

The above embodiment is particularly appropriate for electrolytic capacitors, wherein the first electrode is an electrolyte forming or acting as the cathode, and the at least two second electrodes form or act as the anodes. Advantageously, the cathodic potential is on the housing, thereby the first electrode is contactable from the outside of the capacitor via the housing and separately from the at least two second electrodes.

In some embodiments of the capacitor of the invention, the housing is made of an electrically conductive material and forms the at least first electrode, wherein the at least two second electrodes are electrically contactable from the outside of said housing by at least one feedthrough, wherein each of the at least two second electrode are electrically insulated from each other electrode and from the housing.

In some embodiments of the capacitor of the invention, the housing is made of an electrically conductive material and in electrically conductive communication with one electrode of the at least two second electrodes or forms one of the at least two second electrodes, wherein the at least first electrode and one other electrode of the at least two second electrodes are electrically contactable from the outside of the housing by an feedthrough, and the at least first electrode and the one other electrode are electrically insulated from each other electrode and from the housing.

In some embodiments of the capacitor of the invention, the housing, particularly made of an electrically conductive material, comprises a protrusion element, particularly a planar protrusion element, protruding from the inner wall of the housing and extending into the housing, wherein the protrusion element is arranged between two second electrodes having the second polarity. In case of the housing forms the first electrode or the first electrode is formed by an electrolyte being in electrically conductive communication with the housing, the protrusion element may act as an additional cathodic surface. Due to the described arrangement, the equivalent series resistance may be advantageously reduced.

In some embodiments of the capacitor of the invention, the at least first electrode is electrically connected to one or more electrodes having the first polarity inside the housing.

In some embodiments of the capacitor of the invention, at least one of the at least two second electrodes is electrically connected to one or more electrodes having the second polarity inside the housing.

In some embodiments of the capacitor of the invention, the at least first electrode is arranged between the at least two second electrodes. Advantageously, such arrangement facilitates a reduced equivalent series resistance and a more efficient use of the space within the capacitor of the invention.

In some embodiments, the capacitor of the invention comprises two to thirty two second electrodes having the second polarity, particularly three to twelve second electrodes having the second polarity.

In some embodiments, the capacitor of the invention further comprises at least one separator element. Such separator element is particularly configured to provide mechanical protection of the electrodes. In some embodiments, the at least one separator element is arranged between the at least first electrode and the at least two second electrodes. In some embodiments, the at least one separator element is designed as being at least semipermeable for charge carriers, e.g. charge carrier of an electrolyte. In some embodiments, the at least one separator element is formed of expanded or porous polytetrafluoroethylene (Gore), polypropylene, polyethylene, or a mixture of polypropylene and polyethylene.

In some embodiments of the capacitor of the invention, the at least one separator element at least partly encloses the first electrode, at least one of the at least two second electrodes, or both of the at least two electrodes, wherein particularly the at least one separator element for enclosing is glued, welded, sewn or stitched, pressed, clamped, or riveted or by a combination of the aforementioned techniques.

In some embodiments of the capacitor of the invention, the at least one separator element is formed as a bag or pouch. In some embodiments, the at least one separator element is closable by a traction rope, a zipper, a closure lip, or the like.

In some embodiments of the capacitor of the invention, the at least one separator element is characterized by a pretension, particularly such that the separator element is able to self-sustainingly enclose the electrode or electrodes. Accordingly, the at least one separator element is particularly formed of an elastic material.

In some embodiments of the capacitor of the invention, the at least one separator element is formed of a polymeric material, particularly a thermoplastic material. In some embodiments, the at least one separator element is formed of a polymeric film, particularly a thermoplastic polymeric film, wherein the film is particularly shrinkable by heat such that the film or the separator element acts as a shrink wrap.

In some embodiments of the capacitor of the invention, the at least one separator element encloses two or more electrodes of the same polarity, wherein said two or more electrodes of the same polarity are separated by at least one spacer element.

In some embodiments of the capacitor of the invention, the at least one spacer element is electrically conductive.

In some embodiments of the capacitor of the invention, the at least one spacer element is electrically insulating, and particularly made of a synthetic material, or a polymer, particularly polytetrafluorethylene or silicone, a polymer composition, a ceramic, or a glass.

In some embodiments, the capacitor of the invention is designed as an electrolytic capacitor, wherein particularly the at least one first electrode is designed as an electrolyte acting as cathode, and each of the at least two second electrodes is designed as an anode.

In some embodiments of the capacitor of the invention, each of the second electrodes comprises or consists of a valve metal, particularly aluminium, niobium or tantalum, wherein particularly each of said second electrodes is formed out of sintered body, particularly formed out of a tantalum sintered body.

In some embodiments of the capacitor of the invention, each dielectric layer of each of the second electrodes is by formed at different or equal voltages. In some embodiments, the second electrodes have equal or different energy densities and/or capacitances.

In some embodiments, the capacitor of the invention is an electrolytic capacitor in which the at least two second electrodes comprises or are formed by a valve metal, wherein the at least two second electrodes have a dielectric valve metal oxide layer with a thickness of at least 150 nm, preferably of at least 200 nm. Particularly, the valve metal is aluminium or tantalum.

In some embodiments of the capacitor of the invention, each of the second electrodes is electrically conductively connected with, particularly joined with, an electrically conductive connection element, particularly a pin or a wire. Preferably, the electrically conductive element is joined to the respective electrode during formation of the electrode, e.g. sintering of an electrode, wherein an electrode wire is already attached to the electrode material to be sintered.

In some embodiments of the capacitor of the invention, the electrolyte comprises ethylene glycol and boronic acid, particularly in case of the anodes are formed by aluminium. In some embodiments, the electrolyte comprises dimethylformamide, dimethylacetamide and/or gamma-butyrolactone. In some embodiments, the electrolyte comprises manganese (II) nitrate or manganese dioxide, particularly in case of the anodes are formed by tantalum. In some embodiments, the electrolyte comprises tetracyanoquinodimethane, polypyrrole, or poly(3,4-ethylenedioxythiophene).

In some embodiments, the capacitor of the invention is designed as a film capacitor. Particularly, such film capacitor is characterized by an insulating plastic film as dielectric, which may comprise metallisations on both sides of the film forming the electrodes of the capacitor. Alternatively, the electrodes may be formed by separate metal films. Suitable insulating plastic materials for the dielectric include, without being restricted to, polypropylene, polyester, particularly polyethylene terephthalate, polyethylene napthalate, polyphenylene sulphide, polytetrafluoroethylene, polystyrene and polycarbonate.

In some embodiments, the capacitor of the invention is designed as a ceramic capacitor, wherein, the dielectric of the capacitor is formed by a ceramic material. It may be constructed by several alternating ceramic and metal layers, the latter acting as electrodes (also known as multilayer ceramic capacitors=MLCC).

In some embodiments, the capacitor of the invention is designed as a supercapacitor, such as, for example, an electrostatic double-layer capacitor, pseudocapacitor or hydride capacitor.

In some embodiments of the capacitor of the invention, the at least first electrode or one or all of the at least two second electrodes are designed as:
- a film,
- a sintering body,
- an open porous sponge, or
- a metal deposition on the dielectric by, for example, metallizing, vaporizing, sputtering, or plasma deposition.

In some embodiments of the capacitor of the invention, the surface of the at least first electrode or one of the second electrodes, particularly of a cathode, is coated so as to increase the surface by, for example, printing, spraying, stamping, or galvanic. In some embodiments, the surface of the at least first electrode or one of the at least two second electrodes, particularly of a cathode, is coated with ruthenium oxide, palladium oxide, titan nitride and/or carbon.

In some embodiments of the capacitor of the invention, the housing comprises or consist of a beaker, particularly a deep drawn beaker, and a lid, or two half shells, wherein particularly one or more feedthroughs may be arranged at or within the beaker, the lid, one half shell, or at or within the interface between the beaker and lid or between the two half shells.

In some embodiments, the capacitor of the invention comprises at one least rounded edge, particularly characterized by a radius in the range of 0.5 mm to 8mm.

In some embodiments, the capacitor of the invention is characterized by one of the following shapes: cuboid, prismatic, cylindrical, or conic, particularly truncated conic.

In some embodiments, the capacitor of the invention is characterized by a nominal voltage in the range of 50 V to 1200 V, particularly by a nominal voltage of 235 V, 250 V, 255 V, 400 V, or 1200 V.

In some embodiments, the capacitor of the invention is characterized a leakage current of less than 2µA/µF (measured 5s after end of charging). In some embodiments, the capacitor of the invention is characterized a decrease of the charging time of less than 20 % after storing 5 days at 85 °C.

In some embodiments of the capacitor of the invention, each of the at least two second electrodes forms a partial capacitance with the at least first electrode or any further first electrode having the first polarity, wherein for each first or second electrode the capacitance is in the range of 100nF to 1000µF, particularly in the range of 1 µF to 600 µF. In case of an electrolytic capacitor, the aforementioned capacitances are anode capacitances.

In some embodiments, the capacitor of the invention is characterized by an energy density of at least 2 J/cm³.

The capacitor is particularly suitable for applying in a device, which delivers at least 20 pulses with peak currents of more than 10 A for therapy, such as for example, a cardioverter defibrillator.

According to claim 14, an electronic device is provided, which comprises
- the capacitor of the invention,
- an electronic module, and
- a power source, particularly a battery,
wherein
the capacitor of the invention is electrically connected to the electronic module and/or to the power source.

In some embodiments, the electronic device of the invention is designed as a pacemaker, neurostimulator, a muscle stimulator, or a cardioverter defibrillator, particularly a subcutaneous cardioverter defibrillator.

In some embodiments, the electronic device of the invention is designed as an external defibrillator, particularly an external defibrillator wearable by a patient (e.g. LifeVest).

In some embodiments of the electronic device of the invention, the electronic module comprises a pulse generating unit or circuit, particularly a pacing unit or circuit and/or a shock unit or circuit, being able to generate an electric pulse.

In some embodiments of the electronic device of the invention, the electronic module, particularly the pacing unit or circuit, is configured or designed to provide a low voltage pulse, particularly a series of low voltage pulses, for example with a voltage in the range of 1.0 to 7.5 V.

In some embodiments of the electronic device of the invention, the electronic module, particularly the shock unit or circuit, is configured or designed to provide a high voltage pulse, wherein preferably the electronic module is connected to a capacitor, and the capacitor is charged and discharged with the high voltage pulse, e.g. in the range of 100 to 1200V.

In some embodiments, the electronic device of the invention is able to generate a voltage in the range of 0 V to 50 V. In some embodiments, the electronic device of the invention is able to generate a voltage in the range of 0 V to 3000 V.

Further advantages, features and embodiments of the present invention will be explained hereinafter with reference to the drawings, in which:
- Fig. 1: shows a general scheme of the capacitor of the invention;
- Figs. 2 to 5: show different embodiments of the capacitor of the invention.

### Examples:

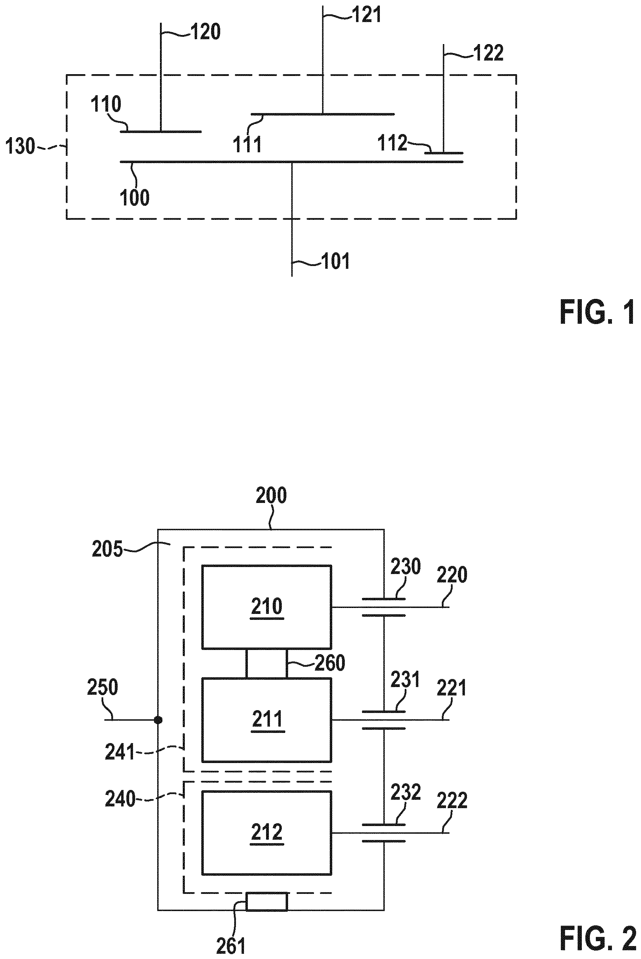

Figure 1 illustrates exemplary the basic structure of the capacitor of the invention. The capacitor has an electrode 100 having a first polarity and, for example, three further electrodes 110, 111, 112 having an opposite polarity. The latter comprise electric connections 120, 121, 122, which are guided through the housing 130 to the outside of the capacitor, where the connections 120, 121, 122 can be contacted with a circuit. Importantly, the connections 120, 121, 122 of the electrodes 110, 110, 112 having the opposite polarity are separated and electrically insulated from each other. The electrode 100 having the first polarity can be electrically connected to the housing and is thereby contactable from the outside of the capacitor and separately from the electrodes having the opposite polarity. Alternatively, the electrode 100 having the first polarity may also comprises an electric connection 101, which is guided separately and electrically insulated from the electric connections 120, 121, 122 of the electrodes 110, 111, 112 having the opposite polarity through the housing 130 to the outside of the capacitor.

Figure 2 illustrates a preferred embodiment of the capacitor of the invention. The capacitor comprises a housing 200, which is optionally filed with an electrolyte 205 forming or acting as a cathode. The housing 200 further comprises anodes 210, 211, 212, which are optionally surrounded by separators 240, 241. For example as depicted in figure 2, two anodes 210, 211 may be enclosed by a common separator 241, while the other anode 212 may be comprised within an own separator 240. The anodes 210, 211, 212 are contactable from the outside of the capacitor by anode wires 220, 221, 222, which are guided by feedthroughs 230, 231, 232 through the housing 200 to the outside of the capacitor. In this embodiment, the cathodic potential is on the housing 200 and can be electrically contacted by an electric connection or port 250. The anodes 210, 211, 212 may be oriented and/or fixed towards each other by a spacer 260. Furthermore, anodes 210, 211 may be arranged within a common separator 241. An additional spacer 261 may also serve for supporting an anode 212 against the housing 200.

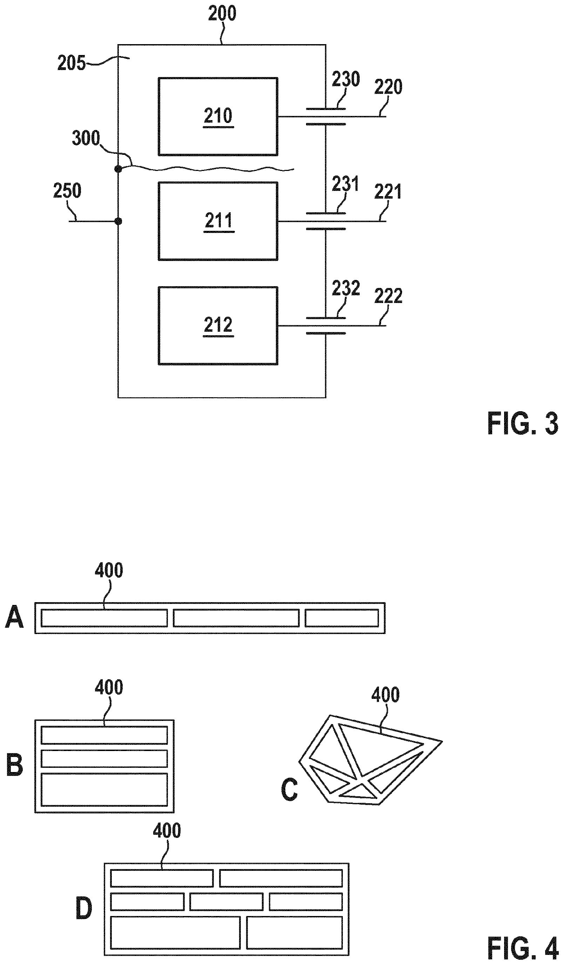

Figure 3 demonstrates a further embodiment of the capacitor of the invention. The reference signs of this figure have the same meaning as in figure 2. This embodiment is particularly characterized by an improved coupling between anodes 210, 211 and the cathode 205 and cathodic surfaces 300, 200, which is particularly suitable for reducing the Equivalent Series Resistance (ESR) of electrolytic capacitors. This is basically realized by arranging an additional cathode or cathode surface 300 between the anodes 210 and 211, wherein the additional cathode surface 300 is electrically connected to at least one of the cathodes 205 and other cathodes surfaces 200, which is in this case the housing 200 having the cathode potential.

Figure 4 shows appropriate arrangements of electrodes 400 for the capacitor of the invention. Those arrangements are particularly suitable for electrolytic capacitors, wherein the electrodes 400 are arranged side by side (A), one above the other (B), in sectors (C) or in form of a brickwork (D).

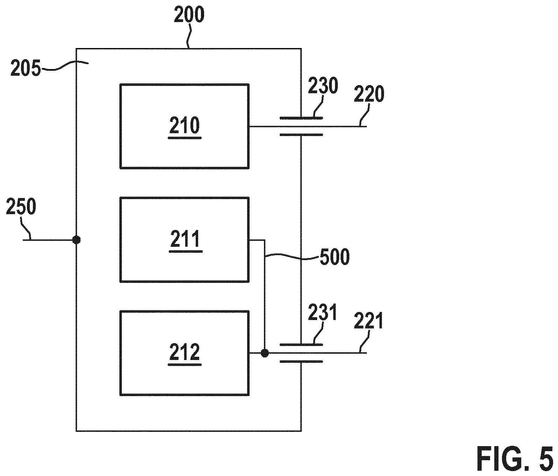

Figure 5 illustrates another embodiment of the capacitor of the invention, wherein anodes 211 and 212 internally, e.g. inside the housing 200 of the capacitor, are connected via a common anode wire 500, which is in turn guide by a feedthrough 231 through the housing 200to the outside of the capacitor. On the other hand, anode 210 is electrically contacted from the outside of the capacitor by its own anode wire 220, which is also guided through the housing 200 by a separate feedthrough 230.

## Claims

1. Capacitor comprising
- at least a first electrode (205, 200, 300) having a first polarity,
- at least two second electrodes (210, 211, 212) having a second polarity opposite to said first polarity, and
- a housing (200) accommodating said at least first electrode (205, 200, 300) and said at least two second electrodes (210, 211, 212),
**characterized in that**
each of said at least first electrode (205, 200, 300) and said at least two second electrodes (210, 211, 212) are electrically contactable separately from each other from the outside of said housing (200).

2. The capacitor according to claim 1, **characterized in that** said at least first electrode (205, 200, 300) or said at least two second electrodes (210, 211, 212) are electrically contactable form the outside of the said housing (200) via a feedthrough (230, 231, 232) extending through said housing (200), wherein particularly said feedthrough (230, 231, 232) comprises an insulating body made of a glass, a ceramic or a polymer, particularly an LCP or an epoxy resin, or a composite thereof.

3. The capacitor according to claim 1 or 2, **characterized in that** said housing (200) is made of an electrically conducting material, and
- said housing (200) is in electrically conductive communication with said at least first electrode (205, 300), and said at least two second electrodes (210, 211, 212) are electrically contactable from the outside of said housing (200) by at least one feedthrough (230, 231, 232), wherein each of said at least two second electrodes (210, 211, 212) are electrically insulated from each other electrode and from said housing (200), or
- said housing (200) forms said at least first electrode, and said at least two second electrodes (210, 211, 212) are electrically contactable from the outside of said housing (200) by at least one feedthrough (230, 231, 232), wherein each of said at least two second electrode (210, 211, 212) are electrically insulated from each other electrode and from said housing (100).

4. The capacitor according to any one of the preceding claims, **characterized in that** said housing (200) comprises a protrusion element (300), particularly a planar protrusion element, protruding from an inner wall of said housing (200) and extending into said housing (200), wherein said protrusion element (200) is at least partly arranged between two second electrodes (210, 211, 212) having said second polarity.

5. The capacitor according to any one of the preceding claims, **characterized in that** said housing (200) is hermetically sealed, and particularly resistant against a pressure difference of at least 2 bar(a), preferable a pressure difference in the range of 2 bar(a) to 10 bar(a).

6. The capacitor according to any one of the preceding claims, **characterized in that** at least one of said at least two second electrodes (210, 211, 212) is electrically connected to one or more electrodes having said second polarity inside said housing.

7. The capacitor according to any one of the preceding claims, **characterized in that** said at least one first electrode (205, 300) is arranged between said at least two second electrodes (210, 211,212).

8. The capacitor according to any one of the preceding claims, comprising two to thirty-two second electrodes having said second polarity, particularly three to twelve second electrodes having said second polarity.

9. The capacitor according to any one of the preceding claims, further comprising at least one separator element (240, 241), particularly arranged between said at least first electrode (205, 200, 300) and said at least two second electrodes (210, 211, 212) or between said at least two second electrodes (210, 211, 212), wherein particularly said at least one separator element is designed as being at least semipermeable for charge carriers.

10. The capacitor according to claim 9, **characterized in that** said at least one separator element (241) encloses two or more electrodes (210, 211) of one polarity, wherein said two or more electrodes (210, 211) of the same polarity are separated by at least one spacer element (260).

11. The capacitor according to claim 10, **characterized in that** said at least one spacer element (260, 261) is electrically insulating, and particularly made of a synthetic material, or a polymer, particularly polytetrafluorethylene or silicone, a polymer composition, a ceramic, or a glass.

12. The capacitor according to any one of the preceding claims, **characterized in that** said capacitor is designed as
- an electrolytic capacitor, wherein particularly said at least one first electrode (205) is designed as an electrolyte acting as cathode, and each of said at least two second electrodes (210, 211, 212) as is designed as an anode, or
- a film capacitor, or
- a ceramic capacitor, particularly a multi-layer ceramic capacitor, or
- a supercapacitor.

13. The capacitor according to claim 12, **characterized in that** said each of said second electrodes (210, 211, 212) comprises or consists of a valve metal, particularly aluminium, niobium or tantalum.

14. An electronic device, particularly a medical device, comprising,
- a capacitor according to any one of claim 1 to 13,
- an electronic module, and
- a power source, particularly a battery,
wherein said capacitor is electrically connected to said electronic module and/or said power source.

15. The electronic device, **characterized in that** said electronic device is a pacemaker, neurostimulator, a muscle stimulator, or a cardioverter defibrillator, particularly a subcutaneous cardioverter defibrillator.
